Europäisches Patentamt

European Patent Office

Office européen des brevets

(19)

(11) Publication number: **0 195 872**
**A2**

## EUROPEAN PATENT APPLICATION

(12)

(21) Application number: 85402227.4

(22) Date of filing: 18.11.85

(51) Int. Cl.⁴: **C 12 P 1/06,** C 07 G 11/00
// (C12P1/06, C12R1:465)

(30) Priority: 27.03.85 JP 60833/85

(43) Date of publication of application: 01.10.86
**Bulletin 86/40**

(84) Designated Contracting States: **AT BE CH DE FR GB IT LI
LU NL SE**

(71) Applicant: **THE KITASATO INSTITUTE,
9-1 Shirogane 5-chome Minato-ku, Tokyo (JP)**

(72) Inventor: **Komiyama, Kanki, 104,
Sanraizu-gumyoji 2-3-301, Mutsugawa, Minami-ku
Yokohama-shi (JP)**
Inventor: **Funayama, Shinji, 7-10-18-301, Nagatsuda
Midori-ku, Yokohama-shi (JP)**
Inventor: **Umezawa, Iwao, 6-22-8, Komagome
Toshima-ku, Tokyo (JP)**

(74) Representative: **Ahner, Francis et al, CABINET
REGIMBEAU 26, avenue Kléber, F-75116 Paris (FR)**

(54) Novel cell-cidal antibiotic 82-85-8A and its production.

(57) The invention relates to a novel antibiotic 82–85–8A
having antimicrobial and cell-cidal activity. This antibiotic is
produced by a microorganism belonging to genus Strep-
tomyces. The invention also concerns a production process
thereof.

EP 0 195 872 A2

# NOVEL CELL-CIDAL ANTIBIOTIC 82-85-8A AND ITS PRODUCTION

This invention relates to novel antibiotic 82-85-8A having antimicrobial and cell-cidal activity, and its production.

Prior known peptide lactone series antibiotics produced by genus $\underline{Streptomyces}$ are monamycin-$G_1$, -$G_2$ -$G_3$, -$H_1$, -$H_2$ and -I [J. Chem. Soc. (c), 526 - 537 (1971)].

We have found that a $\underline{Streptomyces}$ isolated from soil sample obtained in Kanagawa-ken, Japan produces antibiotic substance having strong antibacterial activity against Gram positive bacteria and growth inhibitory activity against HeLa $S_3$ cells. An active substance has been isolated from the cultured broth and purified to determine physico-chemical properties as hereinbelow illustrated. A substance having such physico-chemical properties has never been known among prior known antibiotic substances and hence it was designated as 82-85-8A.

An object of the present invention is to provide a novel antibiotic 82-85-8A or its salt.

Another object of the present invention is to provide a process for production of antibiotic 82-85-8A which comprises culturing antibiotic 82-85-8A producing microorganism belonging to genus Streptomyces, accumulating antibiotic 82-85-8A in a cultured broth and isolating the thus imperted antibiotic 82-85-8A therefrom, or if required changing to salt thereof.

Antibiotic 82-85-8A producing microorganism belongs Streptomyces, and Streptomyces strain 82-85 isolated by the present inventors is illustrative only.

Taxonomical properties of the said strain is illustrated hereinbelow.

a. Morphology :

An observation on Waksman agar plate medium at 27°C for 14 days culture of a strain 82-85 is as follows.

Hyphal growth. Substrate mycelia are not splitted. Aerial mycelia are irregularly branched and not whirlly branching. No sporangia on the top of aerial mycelia. Straight lined spore chain linked with over 20 spores. Spore form is elliptical to cylindrical with smooth surface of 0.9 - 1.4 μm in minor axis.

b. Growth condition on various media :

Growth condition on various media at 27°C for 14 days culture illustrated as follows.

| Medium | Growth | Reverse side | Aerial mycelia | Soluble pigment |
|---|---|---|---|---|
| Yeast-maltose agar (ISP medium 2) | medium | pinkish white | pale orange | orcher |
| Oatmeal agar (ISP medium 3) | good | pale brown | pale orange | orcher |
| Starch-inorganic agar (ISP medium 4) | good | pale orange | pale orange | light orcher |
| Glycerol-asparanine agar (ISP medium 5) | good | light yellow | white | light yellow |
| Peptone-yeast-iro agar (ISP medium 6) | good | yellowish brown | gray | light brown |
| Tyrosine agar (ISP medium 7) | good | dark brown | pale yellow | brown |

c. Physiological properties:

(1) Growth temperature: 20 - 37°C, optimal growth

temperature: appro x 27°C

(2) Liquefaction of gelatine (glucose-peptone-gelatine

medium): negative

(3) Hydrolysis of starch (starch-inorganic salt agar

medium): positive

(4) Coagulation of skim-milk and peptonization

(10% skim-milk medium): negative

(5) Formation of melanine pigment (peptone-yeast-iron agar

medium and tyrosine agar medium): negative

(6) Formation of $H_2S$ (peptone-yeast-iron agar medium): negative

(7) Tyrosinase reaction (tyrosine agar): negative

d. Utilization of carbon sources:

Assimilation of carbon sources was observed on Pridham-Gottlieb agar medium at 27°C for 1 - 2 months.

| | |
|---|---|
| L-arabinose | + |
| D-xylose | + |
| D-mannitol | + |
| L-rhamnose | + |
| sucrose | + |
| inositol | + |
| D-fructose | + |
| raffinose | - |

e. Composition of cell wall:

According to analysis by the method of Becker et al. [Appl. Microbiol., 13, 236 - 243 (1965)], LL-type diamino-pimelic acid was detected.

Referring to the above taxonomical properties, the strain 82-85 belongs to genus Streptomyces, and the strain has been deposited in Fermentation Research Institute as FERM-P No. 8140, on 14 March 1985.

In general, Streptomyces is easily to mutate and a mutant strain can be obtained by natural or artificial mutation technique such as ultraviolet or X-ray irradiation or by means of treating with mutagenic agent using N-methyl-N'-nitro-N-nitrosoguanidine and ethyl methanesulfonate. These artificial or natural mutant of the strain 82-85 belonging to genus Streptomyces which can produce antibiotic 82-85-8A is included to use in the present invention.

In the present invention, a strain belonging to genus Streptomyces which can produce antibiotic 82-85-8A, is cultured in a suitable medium. Conventional culture medium for Strepto-myces can be used. Medium to be used is a nutrient medium

0195872

containing assimirable carbon sources, digestible nitrogen sources and, if required, inorganic salt. Examples of assimilable carbon sources are glucose, molasses, starch, dextrin, glycerin or organic acid. Digestible nitrogen sources are, for example, organic nitrogen sources such as peptone, meat extract, yeast extract, dry yeast, soy bean powder, corn steep liquor, cotton seed cake, casein, soy bean protein hydrolysate, amino acid or urea, and inorganic nitrogen source such as nitrate or ammonia. Inorganic salt such as sodium salt, potassium salt, calcium salt or magnesium salt is added if required. Other trace nutrient, growth factor or precursor of the antibiotic 82-85-8A can also be added in a medium.

Conventional culture is aeration culture under shaking or submerged condition. In an industrial condition, submerged aeration culture is preferable. A pH of medium is preferably at neutral condition and culturing temperature is generally 24 - 30°C, preferably at 27°C. Culturing time in liquid medium is generally 3 - 6 days for antibiotic accumulation. When an amount of antibiotic reaches maximum in a culture medium, culture can preferably be terminated. Culturing conditions such as composition of culture medium, temperature, agitation speed, aeration rate can be controlled according to a condition such as strain and others. Anti-foamer such as silicon oil, vegetable oil or surface active agent can also be added.

The thus produced antibiotic 82-85-8A is mainly accumulated in culture filtrate, and so cultured medium is filtered after adding filter-aid such as Celite (trade anme) or Hyfrosuper-cell (trade name), or is centrifuged to separate mycelia and cultured filtrate.

Isolation of antibiotic 82-85-8A can be done by applying the nature of the antibiotic that it is insoluble in hexane and water, and is soluble in alcohol such as methanol or ethanol, chloroform and dichloromethane, and it is an weak basic substance.

Antibiotic 82-85-8A can be extracted from cultured filtrate with water immiscible organic solvent such as chloroform, dichloromethane or butyl acetate. Organic layer is washed, if required, with diluted ethylene diamine tetraacetate solution for removal of metallic ions and dehydrated by adding dehydration reagent such as anhydrous sodium sulfate or anhydrous magnesium sulfate. Dehydrated organic layer is concentrated to remove organic solvent. In order to avoid decomposition of antibiotic 82-85-8A, temperature at concentration should be preferably below 60°C. Organic solvent such as hexane or petroleum ether is added in the concentrated residue to precipitate the antibiotic. Precipitate is washed several times with hexane and filtered or centrifuged to obtain crude brown colored antibiotic 82-85-8A.

Further purification can be done by various operations such as difference in solubility of contaminant and the antibiotic, difference in partition for two immiscible liquid layer, or difference in adsorption on adsorbing carrier, thus, among them, chromatographic operation is preferable.

Examples of chromatography are adsorption chromatography using adsorption resin such as silica-gel, alumina, active carbon or HP-20 resin (trade name), reverse phase partition chromatography using silylated silica-gel or octadecyl silylated silica-gel, gel-filtration chromatography using Sephadex LH-20 or Toyopearl (trade names), an ionexchange chromatography using DEAE-cellulose, DEAE-Sephadex or DEAE-toyopearl (trade names).

Antibiotic 82-85-8A can be purified by combining or repeating the chromatography, electrophoresis, counter current distribution, ultra-filtration or distillation    For example, crude antibiotic dissolved in small amount of chloroform or benzene is adsorbed in previously packed silica-gel column and eluted with mixed solution of chloroform-methanol, then combined active fraction is concentrated in vacuo.  The thus obtained active substance dissolved in a small amount of methanol is charged on a reverse phase silica-gel column and eluted with mixed solution of methanol-water to purify antibiotic 82-85-8A.

Antibiotic 82-85-8A is an weak basic substance and is prepared as a salt.  Salt is a pharmacologically acceptable non-toxic salt, for example acid addition salt such as inorganic salt like hydrochloride, sulfate, phosphate or carbonate and organic salt like acetate, propionate, tartrate, citrate, malate, glutamate, aspartate, methanesulfonate or p-toluene sulfonate.  Other known acid addition salts can be included.

Physico-chemical properties of antibiotic 82-85-8A are illustrated as follows.

(a) Elementary analysis: $C_{31}H_{47}N_8O_{10}Cl$ (high resolution

mass spectrometry)

(b) Molecular weight: 726.5 [field desorption (FD) mass spectrometry and calculated from elementary analysis]

(c) Melting point: 162 - 167°C (partially immersed at 160°C, completely melt at 167°C, no browning)

(d) Specific rotation: $[\alpha]_D^{20} = -56°$ ( c = 0.24, methanol)

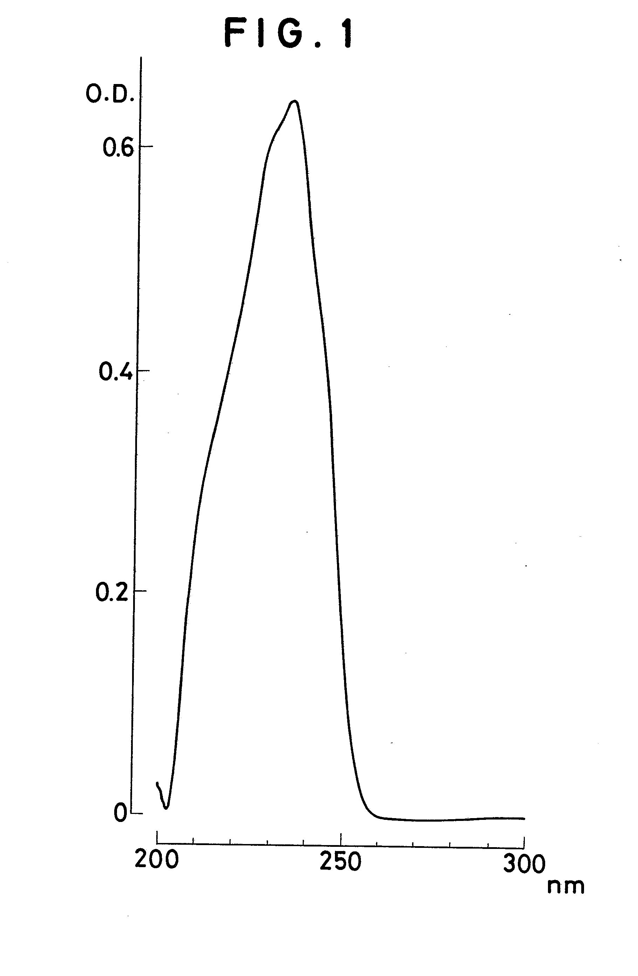

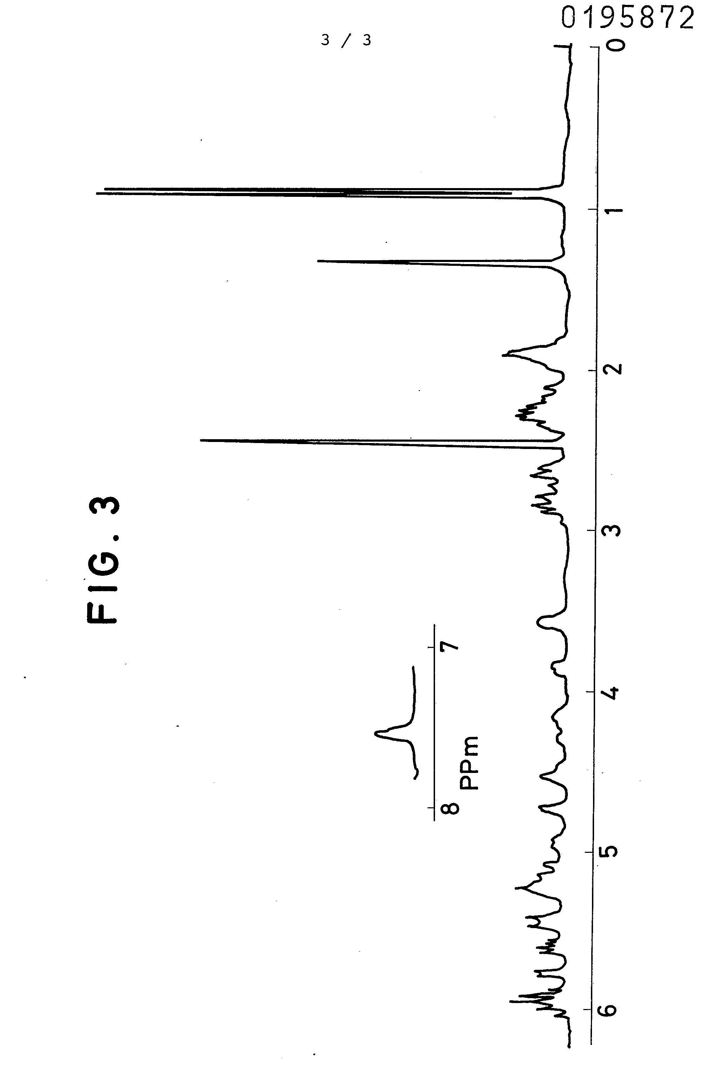

(e) Ultraviolet absorption spectrum: shown in Fig. 1 Maximum absorption in methanol: 226 nm (shoulder), 231 nm, 240 nm (shoulder)

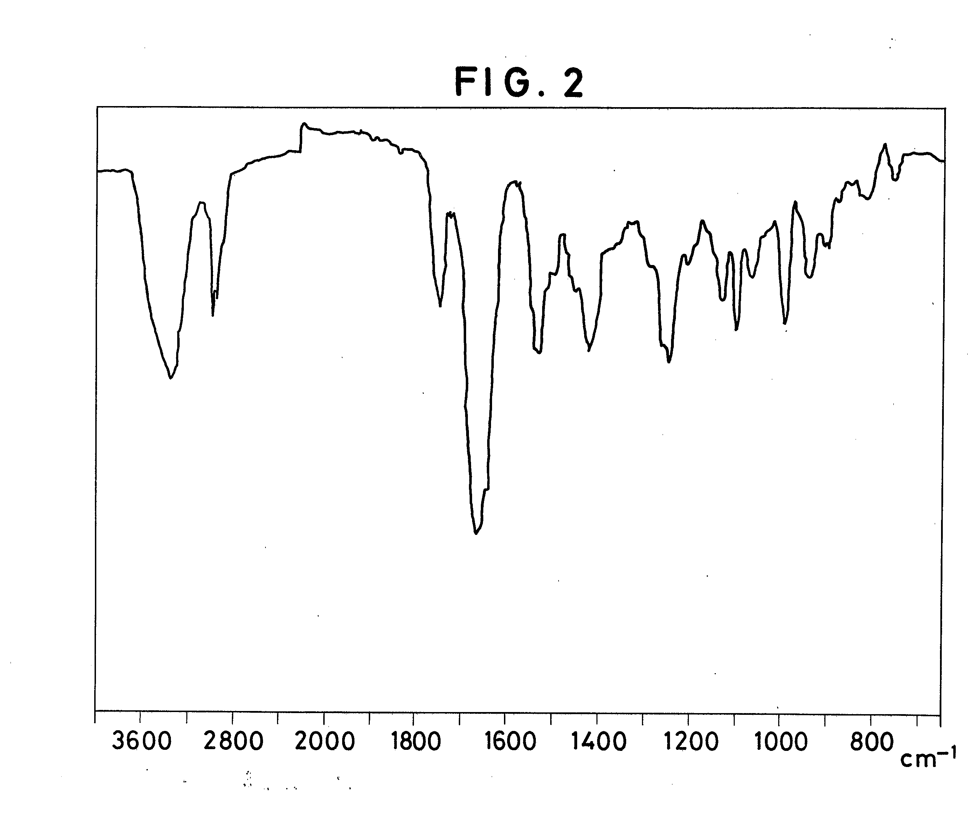

(f) Infrared absorption spectrum (KBr disc): shown in Fig. 2 3340, 1664, 1640, 1530, 1422, 1260, 1244, 1125, 1100, 1067, 992 $cm^{-1}$

(g) solubility:

soluble: chloroform, dichloromethane, ethyl acetate, methanol, ethanol.

slightly soluble: acetone,

insoluble: hexane, water

(h) Color reaction:

positive: iodine, sulfuric acid, Dragendorf reaction

negative: ninhydrin, ferric chloride

(i) Nature: weak basic substance

(j) H-NM spectrum [in $CDCl_3$, inner standard; trimethysilan (TMS) $\delta_{ppm}$]: shown in Fig. 3

(k) Mass spectrum: main mass fragment peaks (EI-MS method):

m/z; 726, 696, 617, 580, 503, 429, 355, 296, 259, 203, 187, 167, 150, 148, 128, 121, 110

(l) Rf-value; silica-gel TLC :

| develop. solvent; | Rf-value; |
|---|---|
| chloroform - methanol (19 : 1) | 0.48 |
| chloroform - acetonitrile (17 : 3) | 0.07 |

(Merck, Kieselgel 60 $F_{254}$ TLC plate)

(m) Stability: stable under an acidic condition at ambient temperature slightly unstable under alkaline condition over pH 10

As illustrated from physico-chemical properties hereinabove, antibiotic 82-85-8A is an water insoluble weak basic peptidelactone antibiotic containing chlorine in its molucule.

Among prior known antibiotic resemble to the present compound, monamycins, namely monamycin-$G_1$, -$G_2$, -$G_3$, -$H_1$, -$H_2$ and -I have been known. Comparing both antibiotics, antibiotic 82-858A has different properties on molecular composition, molecular formula and nuclear magnetic resonance spectrum, and hence antibiotic 82-85-8A is confirmed as novel antibiotic.

| | | Molecular formula | M.W. |
|---|---|---|---|
| Monamycin | $G_1$ | $C_{34}H_{54}N_7O_8Cl$ | 723.5 |
| " | $G_2$ | $C_{33}H_{54}N_7O_8Cl$ | 711.5 |
| " | $G_3$ | $C_{33}H_{54}N_7O_8Cl$ | 711.5 |
| " | $H_1$ | $C_{34}H_{56}N_7O_8Cl$ | 725.5 |
| " | $H_2$ | $C_{34}H_{56}N_7O_8Cl$ | 725.5 |
| " | I | $C_{35}H_{58}N_7O_8Cl$ | 739.5 |

Biological properties of antibiotic 82-85-8A are illustrated as follows.

1) Antibacterial and antifungal activities:

Result of paper disc method (Toyo filter paper, diameter 6 mm) is shown in Table 1.

Table 1.

Minimum inhibitory concentration    ($mcg/ml$)

| | | |
|---|---|---|
| Bacillus subtilis | P C I 2 1 9 | 0.3 |
| Bacillus cereus | | 0.6 |
| Micrococcus luteus | A T C C 9 3 4 1 | 0.3 |
| Staphylococcus aureus | F D A   2 0 9 P | 0.3 |
| Salmonella typhimurium | | >1 0 0 |
| Shigella flexneri | | >1 0 0 |
| Shigella sonnei | E — 3 3 | >1 0 0 |
| Escherichia coli | N I H J | >1 0 0 |
| Klebsiella pneumoniae | P C I   6.0 2 | >1 0 0 |
| Aerobacter aerogenes | | >1 0 0 |
| Proteus burugaris | | >1 0 0 |
| Candida albicans | K F   1 | >1 0 0 |
| Saccharomyces sake | K F·  2 6 | ·>1 0 0 |
| Schizosaccharomyces-pombe | I A M   4 8 6 3 | >1 0 0 |
| Rhizopus japanicus | I A M   6 2 4 1 | >1 0 0 |
| Aspergillus nigar | A T C C   6 2 7 5 | >1 0 0 |
| Alternaria kikuchiana | K F   1 8 5 | >1 0 0 |
| Mucor racemosus | I F O   5 4 0 3 | >1 0 0 |

As illustrated antibiotic 82-85-8A has strong antibacterial activities against Gram positive bacteria.

2) Cell-cidal activity on HeLa $S_3$ cells:

50% cell-cidal value on HeLa $S_3$ cells is approximately 0.2 µg/ml. HeLa $S_3$ cell, $4 \times 10^4$ cells were cultured in a medium for 2 days, and antibiotic 82-85-8A is added therein.

Further, continuing 3 days cultivation, numbers of cells are counted.

3) Toxicity:

$LD_{50}$, ddy mounse, intraperitoneal administration:

approximately 3.8 mg/kg Antibiotic 82-85-8A and its acid addition salt having antibacterial activity and cell cidal activity, are expected as a medicament antibiotic, especially for treatment of Gram positive bacterial infections or as an antitumor agent.

Following examples ellustrate the present invention but are not construed as limiting.

Example 1.

Fermentation :

Streptomyces sp. No. 82085 FERM-P No. 8140, cultured on an agar slant medium containing glucose 1.0%, peptone 0.5%, meat extract 0.5%, NaCl 0.3% and agar 1.2%, at 27°C for 14 days, was inoculated in a sterilized liquid medium (100 ml, pH 7.0) containing glucose 2.0%, peptone 0.5%, meat extract 0.3%, dry yeast 0.3%, NaCl 0.5% and calcium carbonate 0.3% [A-medium] and reciplocally shake cultured (frequency 17 cm, 120 freq./min.) at 27°C for 72 hours to prepare seed culture.

In a 600 lit. tank, A-medium (300 lit.) was prepared, sterilized, aseptically inoculated the seed culture obtained hereinabove and submerged aeration cultured at 27°C, aeration 150 lit./min. for 3 days with agitating to obtain cultured broth (290 lit.).

Example 2.

Extraction of antibiotic:

Cultured filtrate obtained in Example 1 was mixed with hyfrosuper cell (15 kg) and filtered to obtain filtrate
(260 lit.) which was adjusted to pH 6 by adding 6N-HCl.
Ethyl acetate (150 lit.) was mixed with the filtrate, stirred
and the antibiotic 82-85-8A was extracted.  Aqueous layer
was again mixed with ethyl acetate (150 lit.) and stirred.
Combined ethyl acetate layer was concentrated in vacuo up to
approximately 10 lit., then washed with de-ionized water (5
lit.).  Organic layer was dehydrated by adding anhydrous sodium
sulfate, and was distilled off in vacuo to obtain oily brownish substance antibiotic 82-85-8A.

Example 3.

### Silica-gel chromatography of antibiotic:

Oily substance obtained in Example 2 was charged on
a column (95 X 500 mm) of silica-gel 60 (Merck), which was
previously packed with chloroform, and chromatographed by
continuous exchanging elution solvent from chloroform to
methanol.  Eluted fraction showing antibacterial activity
against Micrococcus luteus ATCC 9341 were collected, and
concentrated in vacuo to obtain antibiotic 82-85-8A with approximately 50% purity.

Example 4.

### Isolation of antibiotic by HPLC:

Purification of crude antibiotic 82-85-8A obtained in Example
3 was performed by means of high speed liquid chromatography,
assembled with the following parts.

       Injection pump: TRIROTAR-V (Japan Spectrometry Corp.);

       Detector: UVIDEC-100-V (Japan Sepctrometry Corp.);

       Column: Octadecyl silylated silica-gel in YMC packed column A-324, inner diameter 10 mm X length 300 mm.

             (Yamamura Chem. Inst. Inc.);

Crude antibiotic 82-85-8A (5 mg) dissolved in methanol (300 µl) was injected and eluted with a mixture of water-methanol (3 : 7). Peak showing absorption at 232 nm was collected and concentrated in vacuo to obtain purified antibiotic 82-85-8A.

Example 5.

Isolation of antibiotic by preparative TLC:

Silica-gel 60 $F_{254}$, 20 X 20 cm (Merck) was used for preparative thin layer chromatography. Partially purified antibiotic 82-85-8A (20 mg) dissolved in a small amount of chloroform was spotted on a streak line. The plate was developed with a mixed solvent of chloroform - methanol (19 : 1). Antibiotic 82-85-8A was checked by irradiating with ultraviolet lamp and bund showing antibiotic 82-85-8A was scratched off which was extracted with chloroform to obtain purified antibiotic 82-85-8A (10 mg).

Brief description of drawings :

Fig. 1: ultraviolet absorption spectrum of antibiotic 82-85-8A.

Fig. 2: infrared absorption spectrum of antibiotic 82-85-8A.

Fig. 3: proton nuclear magnetic resonance (NMR) spectrum of antibiotic 82-85-8A.

CLAIMS

1/ Antibiotic 82-85-8A of the following physico-chemical properties or salt thereof :

    a) Elementary analysis: $C_{31}H_{47}N_8O_{10}Cl$ (determined by high resolution mass spectrometry)

    b) Molecular weight: 726.5 (determined by filed desorption mass spectrometry and calculation from elementary analysis)

    c) Melting point: 162-167°C (partially immersed at 160°C, completely melted at 167°C, no browning)

    d) Specific rotation : $[\alpha]_D^{20}$ = -56° (c = 0.24, methanol)

    e) Ultraviolet absorption spectrum (in methanol): shown in Fig.1

    f) Infrared absorption spectrum (KBr): shown in Fig.2

    g) Solubility:

        soluble: chloroform, dichloromethane, ethyl acetate methanol, ethanol,

        slightly soluble: acetone,

        insoluble: hexane, water

    h) color reaction:

        positive: iode, sulfuric acid, Dragendorf, negative: ninhydrin, ferric chloride

    i) Nature: weak basic substance.

2/ A process for production of antibiotic 82-85-8A or salt thereof which comprises culturing antibiotic 82-85-8A producing microorganism belonging to genus _Streptomyces_ until accumulating the antibiotic in culture medium and isolating the thus accumulated antibiotic 82-85-8A, and if required changing to salt thereof.

3/ A process according to claim 2 wherein antibiotic 82-85-8A producing microorganism is _Streptomyces_ sp. No. 82-85, FERM-P No. 8140.

# FIG.1

FIG. 3

# FIG. 2